(19) Europäisches Patentamt
European Patent Office
Office européen des brevets

(11) **EP 3 106 187 A1**

(12) **EUROPÄISCHE PATENTANMELDUNG**

(43) Veröffentlichungstag:
**21.12.2016 Patentblatt 2016/51**

(51) Int Cl.:
*A61M 1/12* *(2006.01)*          *A61M 1/10* *(2006.01)*

(21) Anmeldenummer: **15172410.1**

(22) Anmeldetag: **16.06.2015**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA**

(71) Anmelder: **Berlin Heart GmbH**
**12247 Berlin (DE)**

(72) Erfinder:
• **Wiesener, Constantin**
**14471 Potsdam (DE)**
• **Karch, Dr., Dominik**
**12157 Berlin (DE)**

(74) Vertreter: **Pfenning, Meinig & Partner mbB**
**Patent- und Rechtsanwälte**
**Joachimsthaler Straße 12**
**10719 Berlin (DE)**

(54) **IMPLANTIERBARE HERZPUMPE**

(57) Die Erfindung bezieht sich auf eine Herzpumpe (6) mit einem Förderkanal und mit einer in diesem angeordneten Fördereinrichtung (6b, 6c, 34) für Blut, wobei der Förderkanal wenigstens teilweise in einem Pumpenrohr (6a, 105a) verläuft. An der Herzpumpe, insbesondere an eine Wand des Förderkanals, insbesondere am Pumpenrohr, ist eine erste Sensoreinrichtung (100, 101, 102) zur Messung der Sauerstoffsättigung des Blutes vorgesehen. Auch eine Steuereinrichtung kann mit in die Pumpe integriert sein. Es ergeben sich hierdurch zusätzliche Überwachungs- und Steuerungsmöglichkeiten.

Fig. 6

EP 3 106 187 A1

**Beschreibung**

Dieses Schutzrecht liegt auf dem Gebiet der Medizintechnik und befasst sich insbesondere mit Herzpumpen sowie mit Unterstützungssystemen für Patienten mit ungenügender oder fehlerhafter Herzfunktion.

Implantierbare und körperexterne Pumpen zur Unterstützung und Ergänzung der Herzfunktion bei der Förderung von Blut sind im Stand der Technik inzwischen weit verbreitet und entwickelt. Hierzu gehören auch Steuerungssysteme, die einerseits die Unterstützungsnotwendigkeit ermitteln und den Unterstützungsgrad des Patientenherzens einstellen, wobei anspruchsvolle Aufgaben beispielsweise in der Ermittlung und Einstellung des tatsächlichen Volumenstroms durch die Pumpe liegen.

Derartige Pumpen sind oft als Rotorpumpen mit einer rotierenden Fördereinrichtung mit Förderelementen in Form von Schaufeln ausgebildet, wodurch eine Radialpumpe oder eine Axialpumpe gebildet sein kann. Bei solchen Pumpen wird üblicherweise aus technischen Betriebsparametern der Pumpe, wie beispielsweise der Drehzahl des Rotors, dem Drehmoment oder einer axialen Lagerspannung eines Axiallagers, mithilfe von Kennlinienfeldern der tatsächliche Volumenstrom bestimmt. Zur Steuerung des Unterstützungssystems ist die Berücksichtigung eines weiteren Parameters, nämlich der Sauerstoffsättigung des geförderten Blutes bzw. des Volumenstroms von sauerstoffgesättigtem Blut, hilfreich. Arteriell gemessen (LVAD) hängt dieser Parameter davon ab, wie gut das pulmonale System zur Aufnahme von Sauerstoff funktioniert. Im Falle z. B. eines Lungenödems sinkt die arterielle Sättigung. Im Falle eines RVADs kann die gemischt/zentral venöse Sauerstoffsättigung, gemessen im rechten Vorhof bzw. Ventrikel, zudem zur Erkennung von physiologischen Belastungszuständen verwendet werden.

Aus dem Stand der Technik sind Messeinrichtungen zur Ermittlung der Sauerstoffsättigung im Blut bereits seit langem bekannt. Hervorzuheben ist hierbei das Verfahren der optischen Pulsoxymetrie, bei dem ein Pulsoxymeter im Gewebe befindliches Blut (beispielsweise in einem Finger oder Ohrläppchen) mit einem Lichtstrahl in zwei Wellenlängenbereichen durchleuchtet. Dabei wird der Effekt ausgenutzt, dass oxygeniertes und nichtoxygeniertes Hämoglobin unterschiedliche Absorptionsspektren aufweisen. Es wird der Quotient aus zwei Absorptionswerten in den verschiedenen Wellenlängenbereichen gebildet und somit das Verhältnis der Verteilungsdichte des oxygenierten und des nichtoxygenierten Hämoglobins im Gewebe und damit auch im Blut bestimmt.

Aus der europäischen Patentschrift EP 2 570 143 B1 ist beispielsweise die Verwendung eines Sauerstoffsensors in Verbindung mit einem implantierbaren kardialen Therapiegerät bereits bekannt.

Der vorliegenden Erfindung liegt vor dem Hintergrund des Standes der Technik die Aufgabe zugrunde, eine Einrichtung zur Bestimmung der Sauerstoffsättigung des Blutes eines Patienten mit einem Herzunterstützungssystem derart zu verbinden, dass eine einfache Handhabbarkeit mit einer verlässlichen Messgenauigkeit und guten Reproduzierbarkeit der Messung verbunden wird.

Die Aufgabe wird gemäß Patentanspruch 1 durch eine implantierbare Herzpumpe gelöst. Die Patentansprüche 2 bis 6 bezeichnen Ausgestaltungen der Erfindung. Die Patentansprüche 7 und 8 beziehen sich auf eine Pumpeneinrichtung mit einer implantierbaren Herzpumpe, und der Patentanspruch 9 bezeichnet ein Verfahren zum Betrieb einer Herzpumpe. Die Unteransprüche 10 bis 11 bezeichnen Ausgestaltungen des Betriebsverfahrens.

Beschrieben wird somit eine implantierbare Herzpumpe mit einem Förderkanal und mit einer in diesem angeordneten Fördereinrichtung für Blut. Die Aufgabe wird durch eine an der Herzpumpe, insbesondere an einer Wand des Förderkanals, weiter insbesondere an einem Pumpenrohr, in dem der Förderkanal wenigstens teilweise verläuft, befestigte erste Sensoreinrichtung zur Messung der Sauerstoffsättigung des Blutes gelöst.

Ein Effekt davon ist, dass die Sauerstoffsättigung des Blutes oder, anders ausgedrückt, der Anteil der sauerstoffgesättigten Hämoglobinmoleküle an der Gesamtanzahl der Hämoglobinmoleküle im Blut unmittelbar an der Pumpe erfasst wird. Derartige Pumpen liegen üblicherweise in unmittelbarer Nähe des Herzens und saugen das Blut oft auch unmittelbar aus einer Herzkammer an. Insofern kann sichergestellt werden, dass die erfasste Sauerstoffsättigung des Blutes dem Wert in der entsprechenden Herzkammer entspricht. Zudem kann der Volumenstrom durch die Pumpe ermittelt werden, so dass zusammen mit dem Sauerstoffgehalt insgesamt der Sauerstofffluss durch die Pumpe genau bestimmt werden kann. Die Bauteile der Herzpumpe, an denen die erste Sensoreinrichtung befestigt werden kann, sind zudem konstruktiv unveränderlich, so dass die Randbedingungen zur Sauerstoffmessung reproduzierbar sind. Dies wäre beispielsweise bei einer Anordnung der ersten Sensoreinrichtung unabhängig von der Herzpumpe im Patientenkörper nicht mit derselben Reproduzierbarkeit erfüllt.

Ein weiterer Effekt liegt darin, dass der erfasste, insbesondere der venöse Sauerstoffgehalt des Blutes unmittelbar auch zur Steuerung und/oder Regelung der Herzpumpe verwendet werden kann. Durch die Integration der Sensoreinrichtung in die Herzpumpe ist keine nachträgliche Justage oder Eichung der Messung notwendig, und auch die Verbindungen zu einem Steuergerät der Pumpe sind einfach und zuverlässig herstellbar, insbesondere vor der Implantation.

Eine weitere Ausgestaltung sieht vor, dass die erste Sensoreinrichtung wenigstens eine, insbesondere zwei Strahlungsquellen sowie wenigstens einen, insbesondere zwei Strahlungssensoren aufweist. Die Messung basiert, ebenso wie der Aufbau des Sensors, auf dem bekannten Prinzip der Pulsoxymetrie, das die Sauerstoffsättigung über die Messung einer Lichtabsorption bzw. Lichtreflexion oder -transmission beschreibt. Dem liegt die Erkenntnis zugrunde, dass oxygeniertes und nichtoxygeniertes Hämoglobin unterschiedliche Lichtabsorptionsspektren aufweisen. Demgemäß ist es möglich, durch Messung der Lichtabsorption in wenigstens zwei Wellenlängenbereichen (typisch im 660-Nanometer- und 940-Nanometer-Bereich) die Lichttransmission des Blutes an der ersten Sensoreinrichtung zu messen und die Lichtintensitäten durch Quotientenbildung zueinander ins Verhältnis zu setzen, um den Anteil der oxygenierten Hämoglobinmoleküle an der Zahl der nichtoxygenierten Moleküle oder der gesamten (d. h. oxygenierten und nichtoxygenierten) Hämoglobinmoleküle zu bestimmen. Auf die Gesamtdichte der Hämoglobinmoleküle in dem untersuchten Volumen kommt es dabei in diesem Zusammenhang nicht an.

Die genannte Messmethode ist erprobt und ausreichend zuverlässig und genau, um entsprechende typische physiologische Werte bei einem gesunden und kranken Patienten arteriell (LVAD) und venös ausreichend genau messen zu können.

Als Strahlungsquellen werden üblicherweise Halbleiterdioden (Infrarot-/Rotbereich) und als Strahlungssensor beispielsweise ein Phototransistor verwendet.

Eine weitere Ausgestaltung kann vorsehen, dass der Strahlungssensor die Strahlungsintensität in wenigstens zwei verschiedenen Wellenbereichen unabhängig voneinander erfasst. In dem genannten Fall kann das System zwei Strahlungsquellen in Form verschiedener Dioden, jedoch nur einen einzigen Strahlungssensor mit bekannter Sensitivität in den genutzten Wellenlängenbereichen verwenden. Es ist jedoch auch denkbar, für die Erfassung der Strahlung der beiden Strahlungsquellen unterschiedliche Strahlungssensoren einzusetzen.

Eine weitere Ausgestaltung sieht vor, dass die Verbindungsstrecke zwischen der/den Strahlungsquelle(n) und dem Strahlungssensor quer, insbesondere senkrecht, zur Flussrichtung des Blutes im Förderkanal liegt. Bei einer solchen Konstruktion lassen sich die Strahlungsquellen und die Strahlungssensoren einfach außerhalb der Blutströmung oder am Rande der Blutströmung anordnen.

Eine optimierte Abdeckung des gesamten Blutstroms und eine möglichst gute Nutzung der Absorption werden gemäß einer Ausgestaltung dadurch erreicht, dass die Strahlungsquelle und der Strahlungssensor einander am Umfang des Pumpenrohres gegenüberliegen.

Die Implementierung kann zudem eine zweite Sensoreinrichtung für die Lagerspannung eines magnetischen Axiallagers eines Pumpenrotors und/oder eine Sensoreinrichtung zur Erfassung des auf den Rotor übertragenen Drehmoments und/oder der Drehzahl vorsehen. Das Drehmoment des Pumpenrotors kann beispielsweise aus dem Pumpenstrom, d. h. der Stromstärke eines den Pumpenrotor antreibenden Elektromotors, bestimmt werden. Insbesondere durch gleichzeitige Messung der Lagerspannung und/oder der Drehzahl und/oder des Drehmoments lassen sich Größen wie Volumenstrom und Druckdifferenz über der Pumpe ermitteln, die auch mit physiologischen Parametern des Patienten eng zusammenhängen. Damit können die gewonnenen Informationen über die Sauerstoffsättigung zusammen mit Daten über den Blutstrom zu weiteren aussagekräftigen Daten weiterverarbeitet werden.

Gegebenenfalls ist bei Erfassung einer ausreichenden Anzahl von Parametern auch eine Berechnung der Blutviskosität und damit auch des Hämatokrits im Blut möglich.

Die Überlegungen beziehen sich zudem auf eine Pumpeneinrichtung mit einer Pumpe der oben erläuterten Art sowie einer Steuereinrichtung oder einer Analyseeinrichtung, die sowohl mit der ersten Sensoreinrichtung als auch mit einem dritten Sensor zur Erfassung der Drehzahl eines Pumpenrotors verbunden ist.

Zudem kann bei einer derartigen Pumpeneinrichtung vorgesehen sein, dass die Steuereinrichtung mit einem Sensor für den Volumenstrom und/oder die Erfassung des Pumpenstroms und der Drehzahl eines den Pumpenrotor antreibenden Motors und/oder mit einem Sensor zur Erfassung der Lagerspannung eines den Pumpenrotor lagernden magnetischen Axiallagers verbunden ist.

Werden der Steuereinrichtung sowohl die Messdaten über die Sauerstoffsättigung des Blutes als auch über die Drehzahl der Pumpe und die Axiallagerspannung und/oder ein Drehmoment des Pumpenrotors übermittelt, so kann in der Steuereinrichtung der Volumenstrom des Blutes durch die Pumpe, der Sauerstoffgehalt und somit auch der Sauerstoffstrom durch die Pumpe in den Körper des Patienten berechnet werden, insbesondere wenn bei diesen Patienten die Aortenklappe/Pulmonalisklappe nicht öffnet und somit der Pumpenfluss dem Herzzeitvolumen entspricht. In diesem Fall kann in einer BiVAD Konfiguration sowohl das Herzzeitvolumen als auch die venöse und arterielle Sättigung gemessen werden und somit auf den Sauerstoffverbrauch des Patienten zurückgerechnet werden.

Aus der Sauerstoffsättigung, insbesondere der venösen, können verschiedene physiologische Zustände des Patienten bestimmt werden, wie beispielsweise die körperliche Belastung des Patienten. Entsprechend kann die Pumpe gemäß der körperlichen Belastung und eines angestrebten optimierten Sauerstoffgehalts gesteuert werden.

Es kann auch bei einer Pumpeneinrichtung mit einer Pumpe der oben genannten Art ein Sensor zur Erfassung des Volumenstroms durch die Pumpe vorsehen sein.

Es kann zudem ein Beschleunigungssensor im Bereich der Herzpumpe vorgesehen sein, um die entsprechenden Sauerstoffsättigungsdaten mit einer potenziellen körperlichen Bewegung und der damit einhergehenden physiologischen Belastung des Patienten näherungsweise zur Plausibilisierung abzugleichen.

Die vorliegenden Ausführungen beziehen sich außer auf eine implantierbare Herzpumpe und eine Pumpeneinrichtung der oben erläuterten Art auch auf ein Verfahren zum Betrieb einer implantierbaren Herzpumpe, bei dem der Volumenstrom und die Sauerstoffsättigung durch Sensoreinrichtungen unmittelbar an der Pumpe ermittelt werden und hieraus der Sauerstoffstrom durch die Pumpe laufend bestimmt wird.

Durch die gleichzeitige Bestimmung des Sauerstoffgehalts des Blutes und des Volumenstroms der Pumpe unter denselben Randbedingungen können die ermittelten Werte mit hoher Genauigkeit und Zuverlässigkeit verknüpft werden und beispielsweise der Sauerstoffstrom durch die Pumpe bestimmt werden. Die Energieversorgung für den ersten Sensor, der den Sauerstoffgehalt des Blutes misst, kann unmittelbar von der Energieversorgung der Herzpumpe, beispielsweise der Energieversorgung des Motors oder der übrigen Sensoren, abgeleitet werden. Die Kabel für die Verbindung der Sensoren zu einem Steuergerät können sämtliche Leitungen der an der Pumpe angeordneten Sensoren miteinander bündeln, derart, dass alle Leitungen durch einen gemeinsamen Kabelmantel miteinander verbunden und eingehüllt werden. Hierdurch können Störungen und Wechselwirkungen einzelner Leitungen mit dem Gewebe minimiert werden.

Das erfindungsgemäße Verfahren kann dadurch vorteilhaft ausgestaltet werden, dass die ermittelte Sauerstoffsättigung oder der ermittelte Sauerstoffstrom durch die Pumpe der Steuerung/Regelung der Drehzahl oder des Volumenstroms durch die Pumpe zugrunde gelegt wird. Beispielsweise kann in dem Fall, dass ein geringer Sauerstoffstrom durch die Pumpe gemessen wird, das geförderte Volumen durch Steigerung der Drehzahl zu fördern versucht werden, um den Körper des Patienten möglichst ausreichend mit Sauerstoff zu versorgen.

Es kann zudem vorgesehen sein, dass aus der Sauerstoffsättigung des Blutes und/oder dem Unterstützungsgrad des Patientenherzens ein Indikator ermittelt wird.

Im Folgenden werden anhand von Figuren einer Zeichnung Ausführungsbeispiele gezeigt und nachfolgend erläutert. Dabei zeigt

Fig. 1　schematisch die Ansicht eines Patientenkörpers mit dem Herzen des Patienten und einer VAD (ventricular assist device)-Pumpe,

Fig. 2　in einer dreidimensionalen Ansicht schematisch eine axiale Rotorpumpe,

Fig. 3　in einer schematischen Seitenansicht das Gehäuse einer Axialpumpe mit einem Rotor, einem Antrieb und einem magnetischen Axiallager,

Fig. 4　ein typisches Kennlinienfeld einer Pumpe mit Kennlinien, die eine Zuordnung einer Drehzahl und einer Druckdifferenz zu einem Volumenstrom ermöglichen,

Fig. 5　ein Diagramm gemäß Figur 4, anhand dessen eine Bestimmung der Druckdifferenz über der Pumpe anhand zwei verschiedener Verfahren und der Vergleich der Ergebnisse erläutert werden,

Fig. 6　schematisch den Aufbau einer Pumpe, in deren Pumpenrohr eine Pulsoxymetrieeinrichtung integriert ist, sowie

Fig. 7　schematisch den Aufbau einer Pumpeneinrichtung zur Anwendung des erfindungsgemäßen Verfahrens.

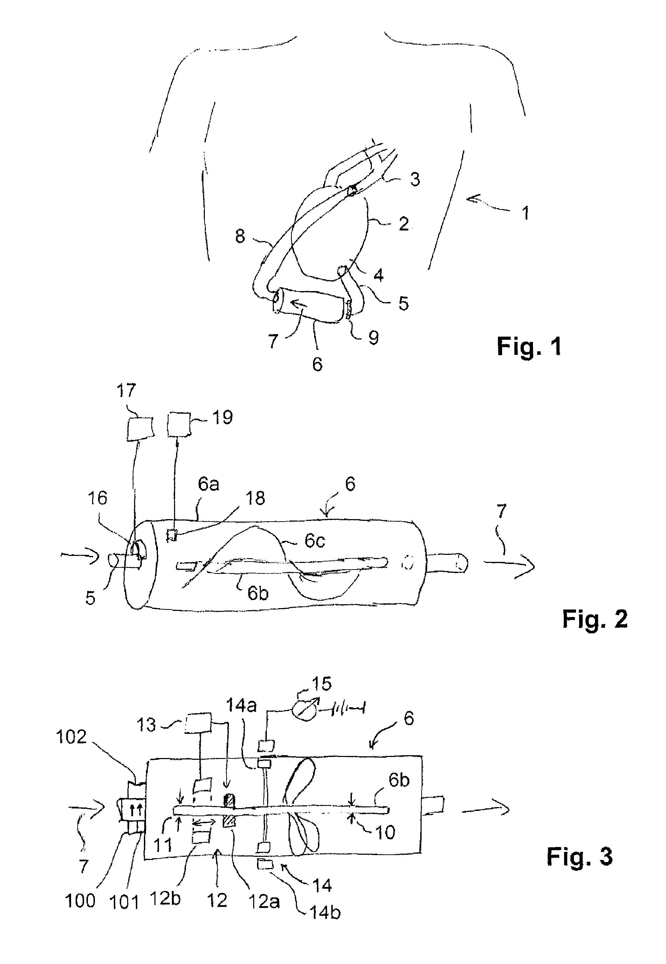

Figur 1 zeigt den Oberkörper eines Patienten 1 mit dem Herzen 2 des Patienten und einem Teil der Aorta 3. An einem Ventrikel 4 des Herzens ist ein Einlassstutzen 5 einer implantierten VAD-Pumpe (ventricular assist device) 6 angeschlossen, die aus dem Ventrikel 4 in Richtung des Pfeils 7 Blut zum Einlass der Pumpe ansaugt und dieses vom Auslass der Pumpe über die Auslasskanüle 8 direkt in die Aorta 3 fördert.

Derartige Pumpen können die Pumpfunktion eines kranken bzw. nicht voll leistungsfähigen Herzens wesentlich unterstützen. Dies kann als vorübergehende Therapie oder auch als Dauertherapie angelegt sein. Als besonders vorteilhaft hat sich dabei die Verwendung von Axialrotorpumpen herausgestellt, die mittels eines schnell rotierenden Rotors im Pumpengehäuse in Axialrichtung 7 Blut fördern. Derartige Pumpen werden üblicherweise durch einen elektromotorischen Antrieb im Bereich des Pumpengehäuses angetrieben, der durch eine mitgeführte Batterie oder einen stationären Stromanschluss gespeist werden kann.

Um physiologische Parameter des Patienten sowie den Betriebszustand der Pumpe ausreichend genau kontrollieren zu können, ist es notwendig, den Volumendurchsatz durch die Pumpe zu ermitteln und zu verfolgen. Hierzu sind grundsätzlich verschiedene Verfahren bekannt, bei denen beispielsweise die Drehzahl des Rotors und die Druckdifferenz über dem Rotor erfasst werden. Auch mittels der Drehzahl der Pumpe ist die Ermittlung des Volumenstroms möglich. Schließlich kann der Volumendurchsatz auch unter Berücksichtigung der Drehzahl des Pumpenrotors und des Drehmoments, das auf den Rotor wirkt, oder der Drehzahl des Pumpenrotors und der Lagerspannung in einem Axiallager des Rotors bestimmt werden.

In Figur 2 ist schematisch eine Axialpumpe 6 mit einer in einem Gehäuse 6a gelagerten Nabe 6b dargestellt, auf der ein oder mehrere Förderelemente 6c, beispielsweise in Form eines umlaufenden schraubenförmigen Förderblattes, befestigt sind. Durch Rotation des Rotors wird in dem Gehäuse 6a die zu fördernde Flüssigkeit bzw. das Blut in Richtung des Pfeils 7 gefördert. Eine Nabe muss nicht notwendig vorgesehen sein, wenn der Rotor in einem magnetischen Axiallager gelagert ist. In diesem Fall ist es nur notwendig, die Förderelemente des Rotors geeignet zu formen, anzuordnen und zu halten.

In Figur 3 ist detaillierter, aber dennoch schematisch, die Lagerung und der Antrieb des Rotors der Pumpe 6 dargestellt. Zunächst ist im Bereich der Nabe 6b ein erstes Radiallager symbolisch dargestellt und mit dem Bezugszeichen 10 bezeichnet, während ein zweites Radiallager ebenso symbolisch dargestellt und mit 11 bezeichnet ist. Das zweite Radiallager 11 kann beispielsweise mit einem magnetischen Axiallager 12 kombiniert, jedoch auch von diesem separat aufgebaut sein.

Das Axiallager 12 ist als regelbares magnetisches Axiallager ausgebildet, wobei ein erster ringförmiger Magnet 12a mit der Nabe 6b oder, wenn der Rotor nabenlos gestaltet ist, mit dem Rotor verbunden ist und mit dieser/diesem rotiert.

Ein zweiter ringförmiger Magnet oder eine ringförmige Anordnung von Einzelmagneten 12b ist ortsfest im Gehäuse 6a der Pumpe 6 angeordnet und umgibt die Nabe 6b oder einen Teil des Rotors. Durch Abstoßung des ortsfesten Magneten 12b oder der ortsfesten Magnetanordnung 12b einerseits und des rotierenden Magneten 12a auf der Nabe 6b wird eine auf den Rotor wirkende Axialkraft aufgenommen, die der Strömungsrichtung 7 der Flüssigkeit durch die Pumpe als Reaktionskraft entgegengesetzt ist.

Das Axiallager 12 weist einen zweiten Sensor zur Erfassung der axialen Position des auf der Nabe befestigten Magneten 12a, beispielsweise mit Wirbelstromsensoren, auf, wobei die Information über die Axialposition an eine Steuerungseinrichtung 13 beispielsweise in Form einer durch die Sensoren gemessenen Spannung ermittelt wird. Damit kann die Regelschleife des Magnetlagers 12 geschlossen werden, und die Steuerungseinrichtung 13 kann die axiale Position des Magneten 12a und damit des Rotors bzw. der Nabe 6b auf eine konstante Größe regeln. Die Kraft, die dabei durch das Axiallager aufgenommen wird, ist anhand der durch die Steuerungseinrichtung 13 aufgebrachten Stromstärke oder der in Wirbelstromsensoren gemessenen Spannung ermittelbar.

Die durch das Axiallager aufgenommene Kraft kann bei einem ungeregelten Lager beispielsweise auch durch Messung der axialen Auslenkung der Nabe gegen die Magnetkraft des Axiallagers bestimmt werden.

In Figur 3 ist schematisch ein Elektromotor 14 dargestellt, der beispielsweise Permanentmagnete 14a, die mit dem Rotor fest verbunden sind, und einen Stator 14b, der mit dem Pumpengehäuse 6a verbunden ist, vorsehen kann. Durch entsprechende Ansteuerung von Statorwicklungen wird somit ein bürstenloser Elektromotor realisiert. Die Stromstärke, mit der der Stator 14b beaufschlagt wird, ist mittels eines Messgeräts 15 messbar, und aus dieser Stromstärke kann in einfacher Weise das erzeugte Drehmoment auf den Rotor ermittelt werden.

Figur 3 zeigt zudem im Bereich des Pumpengehäuses an einem Pumpeneinlassstutzen eine erste Sensoreinrichtung 100, 101, 102, die eine erste Strahlungsquelle in Form einer Diode 100 sowie eine zweite strahlende Diode 101 und einen Fototransistor 102 als Strahlungssensor, der Strahlung beider Dioden detektieren kann, aufweist. Die erste Diode 100 kann beispielsweise bei einer Wellenlänge von 660 Nanometern strahlen, während die zweite Diode 101 bei 940 Nanometern strahlt. Die Strahlungsquellen liegen dem Strahlungssensor diametral am Umfang des Pumpeneinlassstutzens gegenüber. Zumindest Teile des Pumpeneinlassstutzens sind entweder zur Durchstrahlung mit Öffnungen versehen, die durch die Sensoren und die Strahlungsquellen abgedeckt sind, oder aus einem transparenten Material hergestellt. Aus den Absorptionskoeffizienten des Blutes in den einzelnen Wellenlängenbereichen lässt sich die Absorption und damit das Konzentrationsverhältnis der Moleküle feststellen, die die jeweilige Wellenlänge bevorzugt absorbieren (bei der Sauerstoffsättigungsmessung oxygenierte und nichtoxygenierte Hämoglobinmoleküle).

Durch die Quotientenbildung der Konzentrationswerte, die die Sauerstoffsättigung ergibt, findet gleichzeitig eine Normierung statt. Dennoch ist eine Eichung der Sensoreinrichtung sinnvoll, insbesondere wenn bei der Messung ein transparenter Teil des Pumpeneinlassstutzens oder eines Pumpenrohrs durchstrahlt wird.

Die Sättigung ergibt sich aus der Gleichung:

$$S_p O_2 = \frac{HbO_2}{(HbO_2 + Hb)}$$

Dabei ist $S_p O_2$ die Sauerstoffsättigung, Hb die Konzentration des nichtoxygenierten Hämoglobins sowie $HbO_2$ die Konzentration des oxygenierten Hämoglobins. Die Konzentrationen lassen sich durch die jeweilige Strahlungsabsorption bestimmen.

Zurückkommend auf Figur 2 soll erwähnt werden, dass dort ein hydrostatischer Drucksensor 16 am Pumpeneinlassstutzen 5 dargestellt ist, der mit einer entsprechenden Verarbeitungseinrichtung 17 verbunden ist. Auch am Pumpenauslass kann ein Drucksensor vorgesehen sein. Damit kann unmittelbar auch eine Druckdifferenz über der Pumpe ermittelt werden. Zudem ist in Figur 2 ein Temperatursensor 18 innerhalb des Pumpengehäuses 6a dargestellt, der mit einer Bearbeitungseinrichtung 19 verbunden ist. Der Temperatursensor 18 kann auch außerhalb der Pumpe in einem von der geförderten Flüssigkeit durchströmten Bereich angeordnet sein.

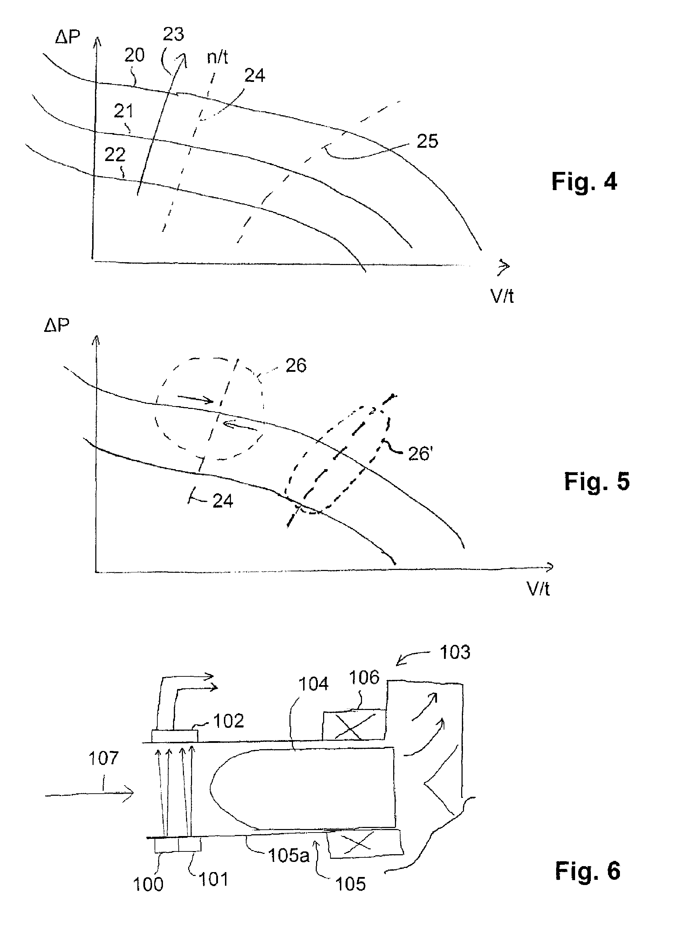

Figur 4 zeigt ein Diagramm, in dem auf der horizontalen Achse der Volumenstrom durch die Pumpe in Volumen pro Zeit gegen die Druckdifferenz über der Pumpe für verschiedene Drehzahlen der Pumpe aufgetragen ist. Dadurch ergibt sich ein Kennlinienfeld, in dem beispielsweise drei Kennlinien 20, 21, 22 für unterschiedliche Drehzahlen dargestellt sind. Der Pfeil 23 zeigt damit tendenziell die Übergangsrichtung zwischen Kurven verschiedener Drehzahlen an.

Damit werden in Form der Kennlinien Betriebspunkte der Pumpe (Druckdifferenz zwischen Ein- und Auslass der Pumpe sowie der durch die Pumpe geförderte Volumenstrom) für verschiedene Drehzahlen der Pumpe dargestellt. Dabei werden zur Kennlinienbestimmung jeweils die Drehzahl und die axiale Lagerposition des Rotors als repräsentative Größe für die Druckdifferenz über dem Rotor aufgenommen. Ersatzweise können auch die Drehzahl und das Drehmoment des Pumpenrotors, gemessen durch den Motorstrom, erfasst werden. Durch entsprechende Regressionskurven können Messpunkte zu Kennlinien interpoliert werden. Im Betrieb der Pumpe kann somit aus den erfassten Betriebsparametern, insbesondere aus der Drehzahl und der axialen Lagerposition des Rotors, beispielsweise der Volumenstrom durch die Pumpe jeweils in bestimmten Bereichen des Kennlinienfeldes bestimmt werden.

Da erkannt wurde, dass für bestimmte Pumpentypen Bereiche im Kennlinienfeld existieren, in denen die Zuordnung zwischen der Lagerposition/Druckdifferenz und dem Volumenstrom bei bekannter Drehzahl nicht eindeutig oder zumindest unscharf ist, kann für bestimmte Bereiche des Kennlinienfeldes ein anderes Verfahren gewählt werden, um den Volumenstrom zu bestimmen. In Abhängigkeit vom Bereich des Kennlinienfeldes, in dem sich der Betriebspunkt der Pumpe befindet, also beispielsweise in Abhängigkeit von der Drehzahl und/oder der Druckdifferenz über der Pumpe, kann somit das entsprechende Ermittlungsverfahren gewählt werden. Auch beim Betrieb der Pumpe anhand des gespeicherten Kennlinienfeldes wird dann in Abhängigkeit von den Betriebsparametern die jeweils passende Auswahl von Betriebsparametern zur Steuerung herangezogen.

In Figur 4 ist beispielsweise zwischen den beiden gestrichelten Linien 24 und 25 ein Teilfeld des Kennlinienfeldes gebildet, in dem der Volumenstrom nicht aus der Drehzahl und der Druckdifferenz über dem Rotor oder zumindest nicht aus diesen Parametern allein bestimmt wird. In dem Feld zwischen den Linien 24 und 25 kann beispielsweise das Drehmoment des Rotors, ermittelt durch den Strom des elektrischen Antriebsmotors des Rotors, und/oder der Absolutdruck am Einlass und/oder Auslass der Pumpe berücksichtigt werden. Diese Größen können allein oder in Kombination mit der Drehzahl ausgewertet werden, es kann jedoch zudem auch die Lagerposition als Indikator für die Druckdifferenz über den Rotor in die Bestimmung des Volumenstroms mit einbezogen werden.

Zudem kann die Fluidtemperatur durch einen Sensor erfasst werden, da insbesondere bei Blutpumpen die Temperatur des Blutes einen starken Einfluss auf die Viskosität und damit auf die Betriebszustände bzw. -parameter der Pumpe hat.

Figur 5 zeigt die Verwendung mehrerer Messverfahren in einem besonderen, vorbestimmten Bereich, der einen Grenzbereich/Übergangsbereich der Kennlinienfelder darstellt und der durch den gestrichelten Kreis 26 angedeutet sein soll. Dieser Bereich liegt in der Nachbarschaft der Trennlinie 24 zwischen den beiden Bereichen des Kennlinienfeldes, die verschiedene Messmethoden zur Ermittlung des Volumenstroms erfordern und ermöglichen. In einem vorbestimmten Bereich um die Trennlinie 24 herum können mehrere Bestimmungsverfahren für den Volumenstrom und /oder die Druckdifferenz über der Pumpe zuverlässig eingesetzt werden, beispielsweise kann der Volumenstrom oder die Druckdifferenz über der Pumpe unter Verwendung unterschiedlicher erfasster Betriebsparameter ermittelt werden. Durch Vergleich der so ermittelten Werte für den Volumenstrom und /oder die Druckdifferenz über der Pumpe kann die Viskosität der geförderten Flüssigkeit, beispielsweise des Patientenblutes, ermittelt werden. Grundsätzlich kann auch ohne konkrete Ermittlung von Volumenstrom und /oder der Druckdifferenz über der Pumpe aus den erfassten redundanten Betriebsparametern die Viskosität ermittelt werden.

Figur 6 zeigt eine Blutpumpe 103 mit einem Rotor 104, der von einem am Pumpengehäuse 105 liegenden Stator 106 elektromotorisch angetrieben wird. Der Rotor weist nicht näher dargestellte Förderelemente auf, die das Blut, das in der Einströmrichtung 107 in das Pumpenrohr 105a einströmt, axial fördern. Das Pumpenrohr 105a ist Teil des Pumpengehäuses 105. In Strömungsrichtung vor dem Rotor 104 ist an dem Pumpenrohr die erste Sensoreinrichtung 100, 101, 102 zur Messung der Sauerstoffsättigung des Blutes befestigt, beispielsweise angeschraubt, angeklebt oder angeklemmt. Die erste Sensoreinrichtung weist eine erste Strahlungsquelle in Form einer Diode 100 auf, sowie eine zweite Diode 101 und einen Fototransistor 102 als Strahlungssensor, der Strahlung beider Dioden detektiert. Die Dioden 100 und 101 können gesondert gleichzeitig oder abwechselnd zur Durchstrahlung des Blutes betrieben werden.

Der Strahlungssensor wird so betrieben, dass er das transmittierte Licht in zwei gesonderten Wellenlängenbereichen jeweils gesondert erfasst, so dass die oben erläuterte Quotientenbildung ermöglicht ist.

Die erste Sensoreinrichtung kann als Ring ausgestaltet sein, an dem die Strahlungsquellen/Dioden und der Strahlungssensor/Fototransistor befestigt sind und der als ganzer auf das Pumpenrohr aufsetzbar und dort verrastbar ist.

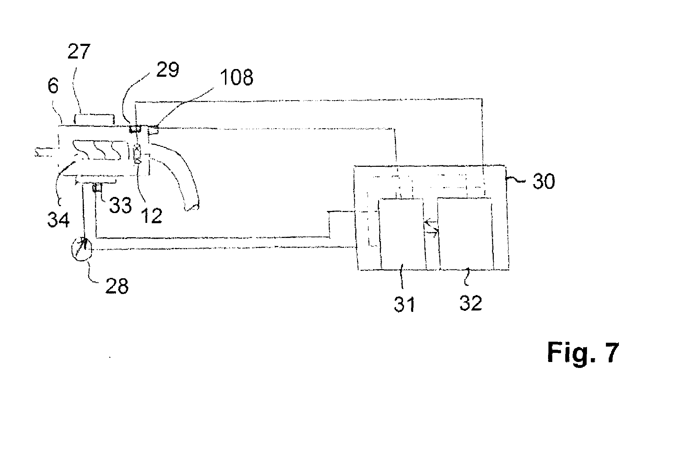

In Figur 7 ist schematisch eine Pumpeneinrichtung dargestellt, die entsprechende Einrichtungen zur Erfassung der Betriebsparameter sowie eine erste Sensoreinrichtung zur Bestimmung der Sauerstoffsättigung des Blutes aufweist. Eine solche Pumpeneinrichtung umfasst eine Pumpe 6 mit einem Rotor 34, ein magnetisches Axiallager 12, einen Sensor 29 für die Lagerspannung sowie einen Motor 27 mit einem außen liegenden Stator, der die Pumpe 6 antreibt. Zudem ist ein Drehzahlsensor 33 sowie ein Stromsensor 28 für den Motorstrom vorgesehen. Die gemessenen Betriebsparameter werden einer Analyseeinrichtung 30 übermittelt, die eine Steuereinrichtung 31 mit einer Speichereinrichtung für Kennliniendaten aufweist. Außerdem weist die Analyseeinrichtung 30 eine Einrichtung 32 zur Ermittlung der Viskosität auf. Diese tauscht Daten mit der Steuereinrichtung 31 aus, so dass jeweils optimierte Kennliniendaten bei der Pumpensteuerung verwendet werden können. Durch die Ermittlung eines Volumenstroms in Verbindung mit der Sauerstoffsättigung des Blutes kann die Versorgung des Patientenkörpers mit Sauerstoff überwacht, beurteilt und gesteuert oder geregelt werden.

Es ist zudem ein Beschleunigungssensor 108 im Körper des Patienten, insbesondere ebenfalls an der Pumpe, angeordnet, so dass eine körperliche Aktivität des Patienten und somit eine physiologische Belastung erkannt und zu der gemessenen Sauerstoffsättigung des Blutes in Beziehung gesetzt werden kann. Der Beschleunigungssensor 108 kann beispielsweise als Halbleiterbauelement ausgebildet sein, das auch mit einer der als Strahlungsquellen dienenden Dioden oder einem als Strahlungssensor dienenden Fototransistor zusammengefasst sein kann.

Die gemessenen Werte der Sauerstoffsättigung und des Volumenstroms können auch der Steuerung eines Herzschrittmachers zugrunde gelegt werden und zu diesem Zweck an die Steuereinheit eines Herzschrittmachers geleitet werden.

Durch die dargestellten Maßnahmen wird die Ermittlung des Volumenstroms durch eine Pumpe, beispielsweise auch unter Verwendung einer aktuell ermittelten Viskosität erheblich verbessert, insbesondere für solche Pumpen, für die eine ausreichende Anzahl von Betriebsparametern in bestimmten Bereichen des Kennlinienfeldes erfassbar ist. Durch die Messung der Sauerstoffsättigung werden verschiedene Messmöglichkeiten am Blut eines Patienten ermöglicht. Die Integration der ersten Sensoreinrichtung in die Pumpe macht die Handhabung, auch bei der Implantation, einfach und die Messung der Sauerstoffsättigung zuverlässig. Die erste Sensoreinrichtung kann nicht nur wie beschrieben als optische Messeinrichtung ausgebildet sein, sondern sich auch jeder anderen bekannten Messmethode bedienen.

**Patentansprüche**

1. Implantierbare Herzpumpe (6) mit einem Förderkanal und mit einer in diesem angeordneten Fördereinrichtung (6b, 6c, 34) für Blut, **gekennzeichnet durch** eine an der Herzpumpe, insbesondere an einer Wand des Förderkanals, insbesondere an einem Pumpenrohr (6a, 105a), in dem der Förderkanal wenigstens teilweise verläuft, befestigte erste Sensoreinrichtung (100, 101, 102) zur Messung der Sauerstoffsättigung des Blutes.

2. Herzpumpe nach Anspruch 1, **dadurch gekennzeichnet, dass** die erste Sensoreinrichtung (100, 101, 102) wenigstens eine, insbesondere zwei Strahlungsquellen (100, 101) sowie wenigstens einen, insbesondere zwei Strahlungssensoren (102) aufweist.

3. Herzpumpe nach Anspruch 2, **dadurch gekennzeichnet, dass** der Strahlungssensor (102) die Strahlungsintensität in wenigstens zwei verschiedenen Wellenbereichen unabhängig voneinander erfasst.

4. Herzpumpe nach Anspruch 2 oder 3, **dadurch gekennzeichnet, dass** die Verbindungsstrecke zwischen der Strahlungsquelle (100, 101) und dem Strahlungssensor (102) quer, insbesondere senkrecht, zur Flussrichtung des Blutes im Förderkanal liegt.

5. Herzpumpe nach Anspruch 2 oder einem der folgenden, **dadurch gekennzeichnet, dass** die Strahlungsquelle (100, 101) und der Strahlungssensor (102) einander am Umfang des Pumpenrohres (6a, 105a) gegenüberliegen.

6. Herzpumpe nach Anspruch 1 oder einem der folgenden, **gekennzeichnet durch** eine zweite Sensoreinrichtung für die Lagerspannung eines magnetischen Axiallagers eines Pumpenrotors, und/oder mit einer Sensoreinrichtung (15, 28) zur Erfassung des auf den Rotor übertragenen Drehmoments und/oder der Drehzahl um den Volumenstrom **durch** die Pumpe schätzen zu können.

7. Pumpeneinrichtung mit einer Pumpe nach einem der Ansprüche 1 bis 6 mit einem Sensor zur Erfassung des Volumenstroms durch die Pumpe.

8. Pumpeneinrichtung nach Anspruch 7, **dadurch gekennzeichnet, dass** die Steuereinrichtung (31) mit einem Sensor (15, 28) für den Volumenstrom und/oder für die Erfassung des Pumpenstroms und Drehzahl eines den Pumpenrotor (34) antreibenden Motors (27) und/oder mit einem Sensor zur Erfassung der Lagerspannung eines den Pumpenrotor lagernden magnetischen Axiallagers (12) verbunden ist.

9. Verfahren zum Betrieb einer implantierbaren Herzpumpe, **dadurch gekennzeichnet, dass** der Volumenstrom und die Sauerstoffsättigung durch Sensoreinrichtungen (15, 28, 29, 33, 100, 101, 102) unmittelbar an der Pumpe (6) ermittelt werden und dass hieraus der Sauerstoffstrom durch die Pumpe laufend bestimmt wird.

10. Verfahren nach Anspruch 9, **dadurch gekenn-**

**zeichnet, dass** die ermittelte Sauerstoffsättigung bzw. der ermittelte Sauerstoffstrom durch die Pumpe (6) der Regelung/Steuerung der Drehzahl/des Volumenstroms durch die Pumpe zugrunde gelegt wird.

11. Verfahren nach Anspruch 9 oder 10, **dadurch gekennzeichnet, dass** aus Sauerstoffsättigung des Blutes und/oder dem Unterstützungsgrad des Patientenherzens ein Indikatorwert ermittelt und signalisiert wird.

Fig. 1

Fig. 2

Fig. 3

Fig. 4

Fig. 5

Fig. 6

Fig. 7

**Europäisches Patentamt**
**European Patent Office**
**Office européen des brevets**

# EUROPÄISCHER RECHERCHENBERICHT

Nummer der Anmeldung

EP 15 17 2410

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br>Y | WO 2011/056980 A2 (WAMPLER RICHARD [US])<br>12. Mai 2011 (2011-05-12)<br>* Zusammenfassung *<br>* Absätze [0042], [0052], [0053] *<br>----- | 1,2,4-11<br><br>3 | INV.<br>A61M1/12<br>A61M1/10 |
| Y | WO 91/08004 A1 (MEDTRONIC INC [US])<br>13. Juni 1991 (1991-06-13)<br>* Zusammenfassung; Abbildungen *<br>* Anspruch 4 *<br>----- | 3 | |
| X | EP 2 564 771 A1 (ECP ENTWICKLUNGSGES MBH [DE]) 6. März 2013 (2013-03-06)<br>* Zusammenfassung; Abbildungen *<br>* Absatz [0053] *<br>----- | 1 | |
| X | US 2013/303831 A1 (EVANS DON W E [US])<br>14. November 2013 (2013-11-14)<br>* Zusammenfassung; Abbildungen *<br>* Absatz [0210] *<br>----- | 1 | |
| X | US 2004/152945 A1 (KANTROWITZ ADRIAN [US] ET AL) 5. August 2004 (2004-08-05)<br>* Zusammenfassung; Abbildungen *<br>* Absatz [0054] *<br>----- | 1 | RECHERCHIERTE SACHGEBIETE (IPC)<br><br>A61M |

Der vorliegende Recherchenbericht wurde für alle Patentansprüche erstellt

| Recherchenort | Abschlußdatum der Recherche | Prüfer |
|---|---|---|
| Den Haag | 14. Dezember 2015 | Walvoort, Bert |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument

&: Mitglied der gleichen Patentfamilie, übereinstimmendes
   Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 15 17 2410

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-12-2015

| Im Recherchenbericht angeführtes Patentdokument | Datum der Veröffentlichung | Mitglied(er) der Patentfamilie | Datum der Veröffentlichung |
|---|---|---|---|
| WO 2011056980 A2 | 12-05-2011 | AU 2010315175 A1<br>CA 2779102 A1<br>CN 102665785 A<br>EP 2496281 A2<br>US 2011118537 A1<br>US 2013303832 A1<br>WO 2011056980 A2 | 17-05-2012<br>12-05-2011<br>12-09-2012<br>12-09-2012<br>19-05-2011<br>14-11-2013<br>12-05-2011 |
| WO 9108004 A1 | 13-06-1991 | AU 643737 B2<br>AU 6913991 A<br>CA 2069004 A1<br>DE 69006369 D1<br>DE 69006369 T2<br>EP 0504270 A1<br>JP H05504074 A<br>US 5067960 A<br>WO 9108004 A1 | 25-11-1993<br>26-06-1991<br>07-06-1991<br>10-03-1994<br>11-05-1994<br>23-09-1992<br>01-07-1993<br>26-11-1991<br>13-06-1991 |
| EP 2564771 A1 | 06-03-2013 | CN 103957781 A<br>DE 112012003687 T5<br>EP 2564771 A1<br>HK 1199699 A1<br>US 2014275720 A1<br>WO 2013034547 A1 | 30-07-2014<br>03-07-2014<br>06-03-2013<br>17-07-2015<br>18-09-2014<br>14-03-2013 |
| US 2013303831 A1 | 14-11-2013 | KEINE | |
| US 2004152945 A1 | 05-08-2004 | US 2004152945 A1<br>US 2014088340 A1 | 05-08-2004<br>27-03-2014 |

Für nähere Einzelheiten zu diesem Anhang : siehe Amtsblatt des Europäischen Patentamts, Nr.12/82

EPO FORM P0461

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2570143 B1